# EUROPEAN PATENT APPLICATION

(11) **EP 3 915 491 A1**
(43) Date of publication of application: **01.12.2021**
(21) Application number: 20790897.1
(22) Date of filing: 24.02.2020
(51) Int. Cl.: A61B 17/04, A61L 17/00, A61F 2/00

(54) **SUTURE, SUTURING APPARATUS, AND APPLICATIONS THEREOF**

(30) Priority: 15.04.2019 CN 201910299526
(71) Applicant: Tongyan (Shanghai) Medical Device Co., Ltd., Shanghai 201203 (CN)
(72) Inventor: YANG, Rui, Shanghai 201203 (CN); YAN, Wei, Shanghai 201203 (CN); XIA, Peipei, Shanghai 201203 (CN); WEI, Zheng, Shanghai 201203 (CN)
(74) Representative: HGF
(86) International application number: PCT/CN2020/076328
(87) International publication number: WO 2020/211526

(57) **Abstract**

Provided are a suture, a suturing apparatus and applications thereof. The suture includes an elongated body, a first cam assembly and a second cam assembly both sleeved on the elongated body. The elongated body includes a first free end and a second free end. The first cam assembly and the second cam assembly each include at least one cam. The cam includes a small end and a large end. The small end of the cam of the first cam assembly faces the first free end of the elongated body and the small end of the cam of the second cam assembly faces the second free end of the elongated body. A cross-sectional area of the small end of the cam is less than a cross-sectional area of the large end of the cam. A wall thickness of the cam tapers along a direction from the small end to the large end. The cam can be secured to the suture by the suture so as not to slip relative to the silk thread when the suture is punctured into tissue and the cam starts to engage with the tissue so as to avoid the problems of offset and stress imbalance on the cam.

## Description

### TECHNICAL FIELD

The present application belongs to the field of medical apparatuses and relates to a suture, a suturing apparatus and applications thereof.

### BACKGROUND

Due to age, environment and other factors, soft tissue and bone capacity of a human face shrink, ligaments start to relax, and the superficial muscular aponeurotic system gradually droops. Facial surface tissue shows saggy cheeks, decreased elasticity, increased wrinkles and deepened folds because of the relaxation of the zygomatic buccal fat pad and the action of gravity. Moreover, with the increase of age, a hormone level inside a human body changes. Collagen loss and facial gland atrophy together with other factors make the human face show a bad characteristic change: aging.

With the progress and development of society, the demand of people for beauty has gradually been increasing. In particular, the demand for facial rejuvenation has been increasing year by year. Currently, in order for the facial rejuvenation to be improved and maintained, a special silk thread made of an absorbable biomaterial is inserted into superficial soft tissue through a minimally invasive surgery. Flabby facial soft tissue is lifted and restored through the excellent lifting of the silk thread and the action of the equal stress distribution.

The targeted lifting for different parts of a face can be implemented, directly acting on the flabby skin. Additionally, the silk thread is made of a biodegradable and bio-absorbable material with good biocompatibility. After a period of time, when the silk thread is degraded and absorbed by the facial tissue, the silk thread can stimulate the regeneration of collagen in subcutaneous tissue. The regenerated collagen forms new retaining ligaments and elastic fibers so that the facial skin can become thickened so as to prevent skin aging.

A medical suture with a barb structure is proposed in US5425747 and US5584859. The suture with a "barb structure" is widely applied in medical cosmetic surgery. When such kind of suture is punctured and embedded into the subcutaneous tissue, the opening direction of the barb is contrary to the puncturing direction. The barb is in a semi-closed status under the oppression of the tissue. After the puncturing is completed, when a main line of the suture is likely to come off, the barb has an engaging effect on the tissue. Moreover, a plurality of barbs work together to generate a large resistance to prevent the suture from coming off.

An improved apparatus for suturing tissue is proposed in US7582105. The improved apparatus is especially advantageous in cosmetic surgery. A suture with a directional cam-type device is provided. The cam-type device may be made into a unidirectional or bidirectional structure to engage with the tissue. Knots are made throughout the silk thread piercing through the cam-type device. A plurality of cam-type devices and a plurality of knots are arranged in sequence on the silk thread. The knots alternate with the cams. One more knot is made before the first cam and one more knot is made after the last cam to limit the cams.

Although the medical suture with a barb structure can lift the flabby skin in some degree, the cam of the suture with a cam-type device has a better engaging effect on the tissue than the barb structure. Moreover, the stimulation and action time of the cam to the skin is also better than the stimulation and action time of the barb structure to the skin. However, the suture only passes through a perforation of the cam and only the knots made before and after the cam are used to limit the forward-backward slip of the cam. When the suture is punctured into the tissue and the cam starts to engage with the tissue, the cam inevitably slips relative to the silk thread in the tissue. Moreover, the centerline of the cam in the tissue may be offset from the suture, making one side of the cam attached to the suture and pulled by the suture. Furthermore, an ordinary cam structure cannot be easily punctured into the tissue in a suturing process, resulting in a bad engaging effect. Thus, a cam with a good engaging effect is urgently needed.

### SUMMARY

An object of the present application is to provide a suture, a suturing apparatus and applications thereof. A cam can be secured to the suture by the suture so as not to slip relative to the silk thread when the suture is punctured into the tissue and the cam starts to engage with the tissue, so as to avoid the problems of offset and stress imbalance on the cam. Additionally, the cam structure can be prepared through thermal expansion, so the preparation cost is relatively low and thus the cam can be industrially produced and applied on a large scale. When the suturing apparatus is used to suture soft tissue and when the suture is lifted at the final puncture position, the problems of stress imbalance on the cam and a failed lift caused by a cam offset can be avoided so that a better lift cosmetic effect can be achieved.

To achieve the object, the present application adopts the technical solutions below.

A first object of the present application is to provide a suture. The suture includes an elongated body, a first cam assembly and a second cam assembly both sleeved on the elongated body. The elongated body includes a first free end and a second free end. The first cam assembly and the second cam assembly each include at least one cam. The cam includes a small end and a large end. The small end of the cam of the first cam assembly faces the first free end of the elongated body and the small end of the cam of the second cam assembly faces the second free end of the elongated body. The cross-sectional area of the small end of the cam is less than the cross-sectional area of the large end of the cam. The wall thickness of the cam tapers along a direction from the small end to the large end.

In the present application, the suture includes the elongated body, the first cam assembly and the second cam assembly. In a suturing process, the bidirectional suturing can be carried out. Moreover, the suture can play a lifting role at the final puncture position so that a better cosmetic effect can be achieved. Additionally, in the present application, the cam can be prepared through thermal expansion of a tubing material of the same material and the preparation method is simple, so the cam can be industrially produced and applied on a large scale.

In the present application, the cross-sectional area of the small end is smaller than the cross-sectional area of the large end. After the cam assembly enters the tissue, the large end can have a better engaging effect on the tissue. In the present application, a space exists between the large end of the cam and the small end of the cam and the wall thickness refers to the thickness of a cam wall surrounding the space. The wall thickness of the cam tapers along a direction from the small end to the large end. The large end with the relatively small wall thickness tightly engages with the tissue so that the cam can be prevented from coming off and affecting the lift effect on the tissue.

In the present application, the maximum wall thickness of the cam is 0.1 mm to 0.9 mm, for example, 0.1 mm, 0.2 mm, 0.3 mm, 0.4 mm, 0.5 mm, 0.6 mm, 0.7 mm, 0.8 mm and 0.9 mm, such as 0.3 mm.

In the present application, the minimum wall thickness of the cam is 0.05 mm to 0.2 mm, for example, 0.05 mm, 0.08 mm, 0.1 mm, 0.12 mm, 0.15 mm, 0.18 mm and 0.2 mm, such as 0.09 mm.

In the present application, the cam is in a trumpet shape containing a hollow portion.

In the present application, the trumpet shape should be construed to conform in appearance to the standard of the generalized trumpet shape. That is a structure whose cross-sectional area is gradually increased from a small end to a large end through a straight line or a curve. The curve includes a concave curve and a convex curve.

The cam may also include a flat segment. The cross-sectional areas of two ends of the flat segment are the same and the flat segment may be cylindrical. The cross-sectional area of the flat segment is less than or equal to the cross-sectional area of the small end of the trumpet shape. In one embodiment, the flat segment is integrated with the elongated body to minimize a slight relative slip between the cam and the silk thread.

The cross-sectional area of the small end or the cross-sectional area of the flat segment is relatively small while the cross-sectional area of the large end is relatively large. When the elongated body drives the cam assembly to puncture the tissue, the order of a single cam entering into the tissue is first the small end and then the large end to facilitate the cam to enter the tissue and minimize the damage to the tissue. Additionally, such an order can also generate a better engaging effect on the tissue.

In the present application, the hollow portion is in an inner trumpet shape. The length of the inner trumpet shape is m along the elongated body, the length of the cam is s along the elongated body, and 1/4s ≤ m ≤ s.

In the present application, the flat segment also includes a hollow inner cylinder (also referred to as an inner flat segment). The length of the inner flat segment is 1 along the elongated body, the length of the cam is s along the elongated body, and 1/3s ≤ 1 ≤ 2/3 s.

In the present application, the length s is 1.5 mm to 3 mm, for example, 1.5 mm, 1.8 mm, 2 mm, 2.2 mm, 2.5 mm, 2.8 mm, 3 mm, such as 2.5 mm. That is, the length 1 is 0.8 mm to 1.6 mm, for example, 0.8 mm, 0.9 mm, 1.0 mm, 1.1 mm, 1.2 mm, 1.3 mm, 1.4 mm, 1.5 mm and 1.6 mm.

In the present application, the hollow portion is in a trumpet shape (it is called the inner trumpet shape in the present application). The hollow portion also includes a flat segment (it is called the inner flat segment in the present application). The inner flat segment is cylindrical. In order for ensuring that after the puncturing is completed, the elongated body directly applies stress to the inner flat segment, avoiding that the elongated body linearly applies stress to the circumferential structure of the large end segment and the small end segment. The length of the inner flat segment is 1, the length of the cam is s along the elongated body, and 1/3s ≤ 1 ≤ 2/3s. When the length 1 of the inner flat segment is less than 1/3s, the relief effect of the inner flat segment on the linear stress is not obvious. When the length 1 of the inner flat segment is more than 2/3s, the proportion of the inner flat segment in the length of the cam is too high, which affects the engaging effect of the cam on the tissue.

In the present application, a trumpet angle of the inner trumpet shape of the hollow portion is α and 20° ≤ α ≤ 120°.

In the present application, the trumpet angle of an opening segment of the trumpet shape of the hollow portion is controlled such that the distance of the inner flat segment of the hollow portion is adjusted to better control the engaging effect. When the trumpet angle α is less than 20°, an opening of the opening segment is relatively small, so the diameter difference between the large end and the small end is relatively small. As a result, after the suture is implanted into the tissue, the large end cannot tightly engage with the tissue and thus a phenomenon that the cam slips off is easy to occur, resulting in the failure of lifting surgery. When α is more than 120°, the opening of the opening segment is relatively large, so the diameter of the large end is too large. As a result, when the suture is implanted into the tissue, a bearing resistance is relatively huge, which is easy to strain the tissue.

In the present application, the first cam assembly and the second cam assembly are symmetric about the midpoint of the elongated body.

In the present application, the first cam assembly and the second cam assembly are provided with the same number of cams.

In the present application, cams of the first cam assembly are equally spaced and cams of the second cam assembly are equally spaced.

In the present application, the first cam assembly is provided with four cams.

In the present application, cams of the first cam assembly are spaced apart at intervals of 3 mm to 10 mm, for example, 3 mm, 4 mm, 5 mm, 6 mm, 7 mm, 8 mm, 9 mm and 10 mm.

In the present application, the second cam assembly is provided with four cams.

In the present application, cams of the second cam assembly are spaced apart at intervals of 3 mm to 10 mm, for example, 3 mm, 4 mm, 5 mm, 6 mm, 7 mm, 8 mm, 9 mm and 10 mm.

In the present application, the cam is secured to the elongated body through hot sealing and/or the cam is limited by the elongated body through a stopper.

In the present application, the cam assembly is secured to the elongated body through hot sealing or the cam assembly is limited by the elongated body through a stopper. In this manner, on one hand, the stability between the elongated body and the cam assembly can be ensured and thus a relative slip can be avoided. On the other hand, after the cam assembly and the elongated body enter the tissue, once the position of the cam is fixed, the elongated body can also be located at a fixed position. In this way, a better lift effect can be achieved.

In the present application, the elongated body is a single stranded silk thread.

In the present application, the elongated body and the cam assembly each are made of a degradable material.

In the present application, the degradable material includes at least one of a synthetic degradable polymer material and natural degradable polymer material.

In the present application, the synthetic degradable polymer material includes any one or a combination of at least two of polylactic acid, L-polylactic acid, DL-polylactic acid, poly(lactic-co-glycolic acid) copolymer, polycaprolactone, poly(3-hydroxybutyrate-co-3-hydroxyvalerate), polyacetylglutamic acid, polyorthoester, polyethylene oxide/polybutene copolymer and poly(lactide-co-carpolactone) copolymer;

In the present application, the natural degradable polymer material includes any one or a combination of at least two of collagen, gelatin, chitosan, hyaluronic acid, sodium alginate and agarose.

A second object of the present application is to provide a suturing apparatus. The suturing apparatus includes the suture described in the first object and metal needles at two ends of the suture.

In the present application, when the tissue is sutured by the suturing apparatus, the suturing apparatus can perform bidirectional suturing and play a lifting role when the suturing is completed so that a better cosmetic or therapeutic effect can be achieved.

In the present application, the suturing apparatus is suitable for wound closure, tissue tension, tissue support, suspension and fixation.

A third object of the present application is to provide applications of the suturing apparatus described in the second object in tissue suturing.

Compared with the existing art, the present application has the beneficial effects below.

In the present application, the cam can be secured to the suture by the suture so as not slip relative to the silk thread when the suture is punctured into the tissue and the cam starts to engage with the tissue, so as to avoid the problems of offset and stress imbalance on the cam. Additionally, the cam structure can be prepared through thermal expansion, so the preparation cost is relatively low and thus the cam can be industrially produced and applied on a large-scale. When the suturing apparatus is used to suture soft tissue and the suture is lifted at the final puncture position, the problems of stress imbalance on the cam and a failed lift caused by a cam offset can be avoided.

### BRIEF DESCRIPTION OF DRAWINGS

FIG. 1 is a schematic view of a suture according to embodiments of the present application.
FIG. 2 is a front view of a cam assembly according to embodiments of the present application.
FIG. 3 is a front view of a cam according to embodiment one of the present application.
FIG. 4 is a sectional view taken along AA' of FIG. 3.
FIG. 5 is a partial enlarged view showing a partial area of FIG. 4.
FIG. 6 is a front view of a cam according to embodiment two of the present application.
FIG. 7 is a sectional view taken along BB' of FIG. 6.
FIG. 8 is a sectional view of a cam according to embodiment three of the present application.
FIG. 9 is a sectional view of a cam according to embodiment four of the present application.

### Reference list

- 1: elongated body
- 2: first free end
- 3: second free end
- 4: first cam assembly
- 5: second cam assembly
- 6: cam
- 7: small end
- 8: large end
- 9: hollow inner cylinder
- 10: cam wall

### DETAILED DESCRIPTION

The solutions of the present application are further described below through the detailed description. Those skilled in the art are to understand that examples described herein are merely used for a better understanding of the present application and are not to be construed as specific limitations to the present application.

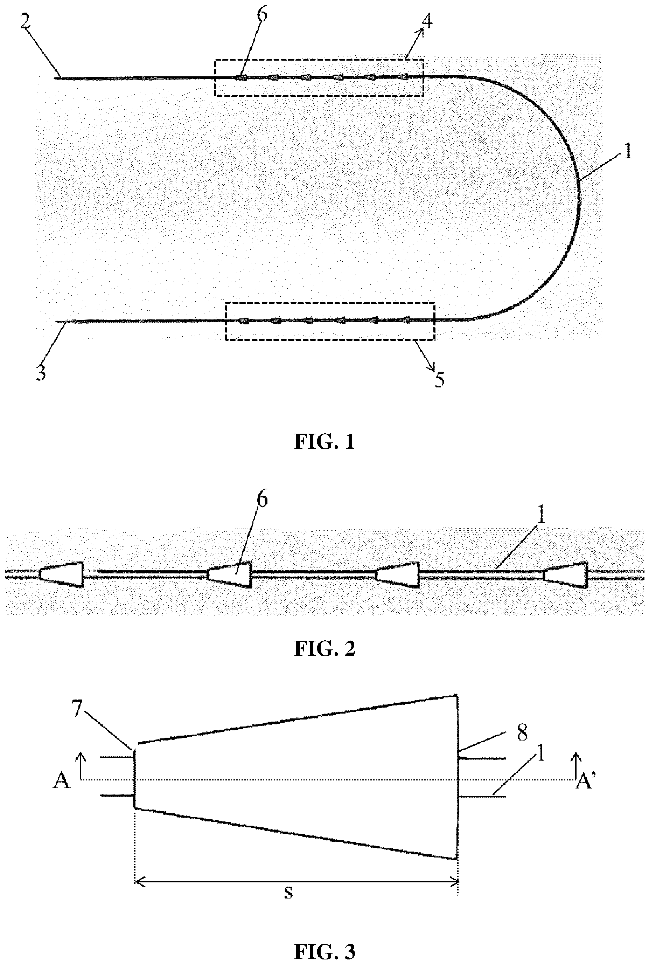

The present embodiment provides a suture. As shown in FIGS. 1 to 2, the suture includes an elongated body 1, a first cam assembly 4 and a second cam assembly 5 both sleeved on the elongated body 1. The elongated body 1 includes a first free end 2 and a second free end 3. The first cam assembly 4 and the second cam assembly 5 each include at least one cam 6. Referring to FIGS. 3 to 4, it can be seen that the cam 6 includes a small end 7 and a large end 8. The small end 7 of the cam of the first cam assembly 4 faces the first free end 2 of the elongated body 1 and the small end 7 of the cam of the second cam assembly 5 faces the second free end 3 of the elongated body 1. A cross-sectional area of the small end 7 of the cam 6 is less than a cross-sectional area of the large end 8 of the cam 6. A wall thickness of the cam 6 tapers along a direction from the small end 7 to the large end 8.

In the present embodiments, the elongated body is a single stranded silk thread. With a relative small diameter and good tenacity, the single stranded silk thread is convenient for the human tissue to be sutured. The diameter, tenacity and the number of layers and other specific conditions of the single stranded silk thread may be adjusted by those skilled in the art based on actual needs. The elongated body, the first cam assembly and the second cam assembly are each made of a biodegradable material. The optional biodegradable material includes any one or a combination of at least two of polylactic acid, L-polylactic acid, DL-polylactic acid, poly(lactic-co-glycolic acid) copolymer, polycaprolactone, poly(3-hydroxybutyrate-co-3-hydroxyvalerate), polyacetylglutamic acid, polyorthoester, polyethylene oxide/polybutene copolymer, poly(lactide-co-carpolactone) copolymer, collagen, gelatin, chitosan, hyaluronic acid, sodium alginate and agarose. The specific selection of the material may be adjusted by those skilled in the art based on actual needs. In the present embodiments, since the suture is left inside the human body after the suturing is completed, the selected biodegradable material can be degraded inside the human body, which does not leave a side effect on the human body.

In the present embodiments, the first cam assembly and the second cam assembly are provided with at least one cam, for example, four cams 6. The number of the cam may be adjusted by those skilled in the art based on actual needs. Cams in the first cam assembly and the second cam assembly are equally spaced at intervals of 3 mm to 10 mm. The specific disposition may be adjusted by those skilled in the art based on actual needs. Moreover, the first cam assembly and the second cam assembly are symmetric about a midpoint of the elongated body, which facilitates enabling a better engaging effect between the tissue and the cam after the suture is punctured into the tissue in a suturing process.

In the present embodiments, the cam includes a small end 7 and a large end 8. The end with a small cross-sectional area is called the small end 7 and the other end with a large cross-sectional area is called the large end 8. The small end of the cam of the first cam assembly 4 faces the first free end 2 of the elongated body and the small end of the cam of the second cam assembly 5 faces the second free end 3 of the elongated body so that during bidirectional puncturing, the small end of the cam assembly faces the free end of the elongated body, which is convenient for puncturing and does not cause much damage to the tissue. The cross-sectional area of the small end of the cam is smaller than the cross-sectional area of the large end of the cam 6. The cross-section areas of the small end and the large end may be adjusted by those skilled in the art based on actual needs. In the present application, a space exists between the large end of the cam and the small end of the cam and a wall thickness refers to the thickness of a cam wall 10 surrounding the space. The wall thickness of the cam tapers along the direction from the small end 7 to the large end 8. The maximum wall thickness of the cam is 0.1 mm to 0.9 mm and the minimum wall thickness of the cam is 0.05 mm to 0.2 mm. The maximum thickness and minimum thickness of the cam wall 10 may be adjusted by those skilled in the art based on actual needs.

In the present embodiments, the elongated body 1, the first cam assembly 4 and the second cam assembly 5 may be secured together through hot sealing or may be integrally manufactured to prevent a relative slip between the cam assembly and the silk thread so that an offset on the cam can be prevented from affecting the engaging effect between the human tissue and the cam. In the present embodiments, the first cam assembly and the second cam assembly may be limited through a stopper. The stopper may be a knot formed by the elongated body or a degradable limiting material. The number of the stopper is at least two more than the number of the cam, which facilitates better securing the cam. A cross-sectional area of the stopper perpendicular to the elongated body is larger than an aperture of the small end of the cam to prevent the stopper from passing through the cam so that the limiting effect is affected. The stopper is a knot or the other degradable limiting material, which may be adjusted by those skilled in the art based on actual needs.

### Embodiment one

In the present embodiment, as shown in FIGS. 3, 4 and 5, the elongated body and the cam are an integral structure. The cam is in a trumpet shape containing a hollow portion. That is, the cross-sectional area of the cam is gradually increased in the direction from the small end 7 to the large end 8 and the wall thickness of the cam wall 10 tapers in the direction from the small end to the large end. The wall thickness of the small end is 0.1 mm and the wall thickness of the large end is 0.05 mm. In the present embodiment, the wall thickness refers to the thickness of the cam wall 10 forming the hollow portion. The hollow portion is in an inner trumpet shape. A length of the inner trumpet shape is m along the elongated body, a length of the cam is s along the elongated body, and m = 1/3s. A trumpet angle of the inner trumpet shape is α and α is 75°.

In the present embodiment, when the suture is punctured into the tissue to suture the tissue, the cam is easy to enter the tissue and can reduce the damage to the tissue since the cross-sectional area of the cam is gradually increased in the direction from the small end to the large end. When the puncturing is completed and the thread is lifted, since the wall thickness of the large end of the cam is relatively small, under the action of the stress, the large end of the cam can better engage with the tissue. Moreover, since the elongated body and the cam are an integral structure, on one hand, the cam can be prevented from slipping off, and on the other hand, the elongated body can be prevented from linearly applying stress to the cam structure so that a better cosmetic or therapeutic effect can be achieved.

### Embodiment Two

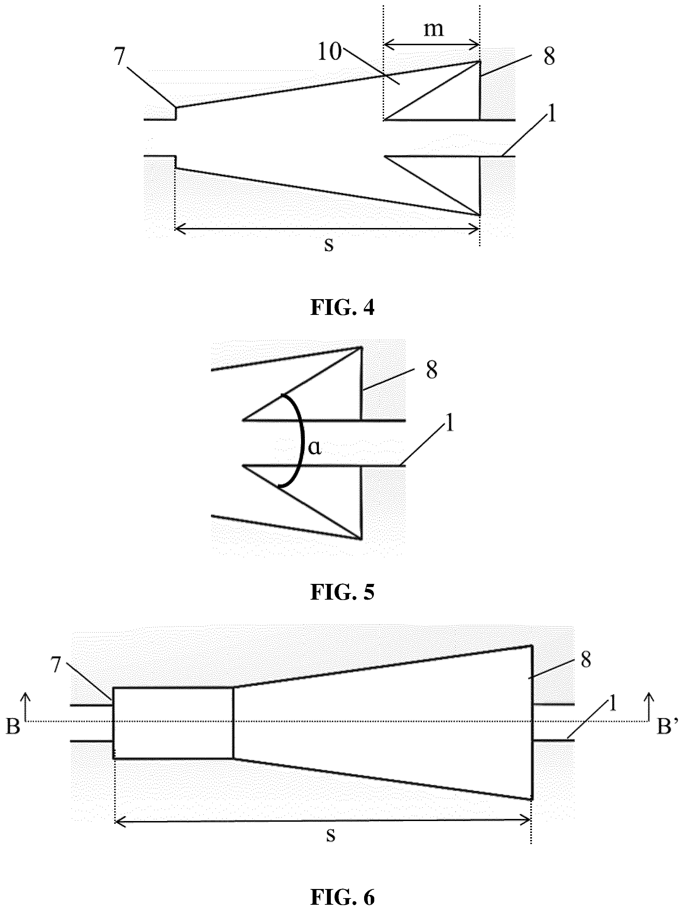

In the present embodiment, as shown in FIGS. 6 to 7, the elongated body and the cam are an integral structure. The cam is in a trumpet shape containing a hollow portion. That is, the cross-sectional area of the cam is gradually increased in the direction from the small end 7 to the large end 8 and the wall thickness of the cam 6 tapers in the direction from the small end 7 to the large end 8. The wall thickness of the small end 7 is 0.9 mm and the wall thickness of the large end 8 is 0.2 mm. In the present embodiment, the wall thickness refers to the thickness of the cam wall 10 forming the hollow portion and a flat segment is also included. The flat segment is cylindrical and the hollow portion is in an inner trumpet shape. A length of the inner trumpet shape is m along the elongated body 1, a length of the cam 6 is s along the elongated body 1, and m = 2/3s. A trumpet angle of the inner trumpet shape is α and α is 45°.

In the present embodiment, when the suture is punctured into the tissue to suture the tissue, the cam is easy to enter the tissue and can reduce the damage to the tissue since the cross-sectional area of the cam is gradually increased in the direction from the small end to the large end. When the puncturing is completed and the thread is lifted, since the wall thickness of the large end of the cam is relatively small, under the action of the stress, the large end of the cam can better engage with the tissue. Moreover, since the elongated body and the cam are an integral structure, on one hand, the cam can be prevented from slipping off, and on the other hand, the elongated body can be prevented from linearly applying stress to the cam structure so that a better cosmetic or therapeutic effect can be achieved.

### Embodiment three

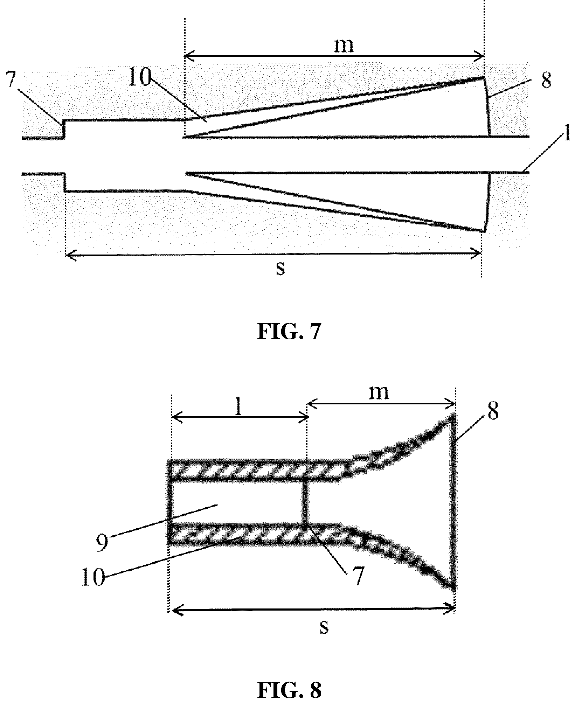

In the present embodiment, as shown in FIG. 8, the cam includes a trumpet shape containing a hollow portion and a flat segment. The flat segment is a cylinder whose diameter is 0.7 mm. The flat segment and the small end 7 of the trumpet shape are equal in diameter and cross-sectional area. That is, the diameter of the small end 7 of the trumpet shape is 0.7 mm and the diameter of the large end 8 of the trumpet shape is 1.3 mm. The cross-sectional area of the trumpet shape is gradually increased from the small end 7 to the large end 8 in a form of a concave curve and the wall thickness of the trumpet shape tapers along the direction from the small end 7 to the large end 8. The wall thickness of the small end 7 is 0.3 mm and the wall thickness of the large end 8 is 0.09 mm. In the present embodiment, the cam wall 10 refers to the wall of the trumpet shape forming the hollow portion and the wall of the flat segment forming the hollow portion. In the present embodiment, the wall thickness refers to the thickness of the cam wall 10 forming the hollow portion. The hollow portion includes an inner trumpet shape and the flat segment also includes a hollow inner cylinder 9 whose diameter is 0.4 mm. A length of the flat segment is 1.3 mm along the elongated body 1 and a length of the cam is 2.5 mm along the elongated body 1. The cross-sectional area of the inner trumpet shape is also gradually increased in direction from the small end to the large end in the form of a concave curve. The radius of the concave curve is 1.75 mm to 1.92 mm. A length of the inner trumpet shape is 1.2 mm along the elongated body. A trumpet angle formed by the tangents of the concave curves on two sides of the small end of the inner trumpet shape is α, and α is 90°.

In the present embodiment, when the suture is punctured into the tissue to suture the tissue, the cross-sectional area of the cam from the small end to the large end is gradually increased. When the puncturing is completed and the thread is lifted, since the wall thickness of the large end of the cam is relatively small, the cam can better engage with the tissue. Moreover, the cross-sectional area of the trumpet shape is gradually increased from the small end to the large end in the form of a concave curve, which can further strengthen the engaging ability between the cam and the tissue. The hollow portion of the cam includes an inner flat segment. The length 1 of the inner flat segment and the length s of the cam along the elongated body meet the requirement of 1/3s ≤ 1 ≤ 2/3s, which ensures that the elongated body directly applies stress to the inner flat segment after the puncturing is completed and prevents the elongated body from linearly applying stress to the circumferential structure of the large end segment and the small end segment. Moreover, the formed trumpet angle is 90°. In this manner, on one hand, an excessively small diameter difference between the large end and the small end can be avoided so that the cam is prevented from slipping off, and on the other hand, an excessively large diameter difference between the large end and the small end can be avoided so that the tissue is not injured from a large resistance arising while the cam is being implanted into the tissue.

### Embodiment four

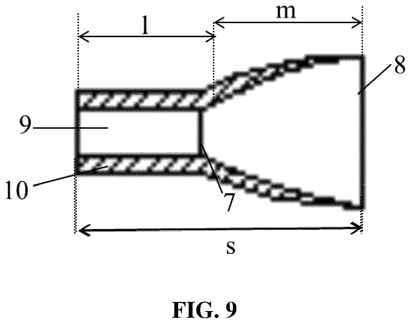

In the present embodiment, as shown in FIG. 9, the cam includes a trumpet shape containing a hollow portion and a flat segment. The flat segment is a cylinder whose diameter is 0.7 mm. The flat segment and the small end 7 of the trumpet shape are equal in diameter and cross-sectional area. That is, the diameter of the small end 7 of the trumpet shape is 0.7 mm and the diameter of the large end 8 of the trumpet shape is 1.3 mm. The cross-sectional area of the trumpet shape is gradually increased from the small end 7 to the large end 8 in a form of convex curve and the wall thickness of the trumpet shape tapers along the direction from the small end 7 to the large end 8. The wall thickness of the small end is 0.5 mm and the wall thickness of the large end is 0.1 mm. In the present embodiment, the cam wall 10 refers to the wall of the trumpet shape forming the hollow portion and the wall of the flat segment forming the hollow portion. In the present embodiment, the wall thickness refers to the thickness of the cam wall forming the hollow portion. The hollow portion includes an inner trumpet shape and the flat segment also includes a hollow inner cylinder 9 whose diameter is 0.4 mm. A length of the flat segment is 1.08 mm along the elongated body and a length of the cam is 2.5 mm along the elongated body. The cross-sectional area of the inner trumpet shape is also gradually increased in direction from the small end to the large end in the form of a convex curve. The radius of the convex curve is 3.5 mm to 4.5 mm. A length of the inner trumpet shape is 1.42 mm along the elongated body. A trumpet angle formed by the tangents of the convex curves on two sides of the small end of the inner trumpet shape is α, and α is 60°.

In the present embodiment, when the suture is punctured into the tissue to suture the tissue, the cross-sectional area of the trumpet shape from the small end to the large end is gradually increased in the form of a convex curve, which can further reduce the difficulty that the cam enters the tissue and the damage to the tissue. When the puncturing is completed and the thread is lifted, since the wall thickness of the large end of the cam is relatively small, the cam 6 can better engage with the tissue. The hollow portion of the cam includes an inner flat segment. The length 1 of the inner flat segment and the length s of the cam along the elongated body meet the requirement of 1/3s ≤ 1 ≤ 2/3s, which ensures that the elongated body directly applies stress to the inner flat segment after the puncturing is completed and prevents the elongated body from linearly applying stress to the circumferential structure of the large end segment and the small end segment. Moreover, the formed trumpet angle is 60°. In this manner, on one hand, an excessively small diameter difference between the large end and the small end can be avoided so that the cam is prevent from slipping off, and on the other hand, an excessively large diameter difference between the large end and the small end can be avoided so that the tissue is not injured from a large resistance arising while the cam is being implanted into the tissue..

The present embodiment also provides a suturing apparatus. The suturing apparatus includes the preceding suture and metal needles at two ends of the suture.

In the present application, metal needles are disposed at two ends of the suture so that bidirectional suturing can be carried out in a suturing process. When the suturing is completed, the elongated body 1 is lifted and a better lifting effect can be achieved so as to be used for suturing tissue.

The applicant declares that the preceding are only the embodiments of the present application and not intended to limit the scope of the present application. Those skilled in the art should understand that any modifications or substitutions easily conceivable by those skilled in the art within the scope of the present application fall within the protection scope and the disclosed scope of the present application.

## Claims

1. A suture, comprising:
an elongated body (1) comprising a first free end (2) and a second free end (3); and
a first cam assembly (4) and a second cam assembly (5) both sleeved on the elongated body (1),
wherein the first cam assembly (4) and the second cam assembly (5) each comprise at least one cam (6),
wherein the cam (6) of the at least one cam (6) comprises a small end (7) and a large end (8),
wherein the small end (7) of the cam of the first cam assembly (4) faces the first free end (2) of the elongated body (1), the small end (7) of the cam of the second cam assembly (5) faces the second free end (3) of the elongated body (1), a cross-sectional area of the small end (7) of the cam (6) is less than a cross-sectional area of the large end (8) of the cam (6), and a wall thickness of the cam (6) tapers along a direction from the small end (7) to the large end (8).

2. The suture of claim 1, wherein a maximum wall thickness of the cam (6) is 0.1 mm to 0.9 mm, preferably 0.3 mm.

3. The suture of claim 1 or 2, wherein a minimum wall thickness of the cam (6) is 0.05 mm to 0.2 mm, preferably 0.09 mm.

4. The suture of any one of claims 1 to 3, wherein the cam (6) is in a trumpet shape containing a hollow portion.

5. The suture of the claim 4, wherein the cam (6) further comprises a flat segment.

6. The suture of claim 5, wherein the hollow portion is in an inner trumpet shape, wherein a length of the inner trumpet shape is m along the elongated body (1), a length of the cam (6) is s along the elongated body (1), and 1/4s ≤ m ≤ s;
preferably, the hollow portion also comprises an inner flat segment, wherein a length of the inner flat segment is 1 along the elongated body (1), and the length of the cam (6) along the elongated body (1) is s, and 1/3s ≤ 1 ≤ 2/3s; and
preferably, a trumpet angle of the inner trumpet shape is α, wherein 20° ≤ α ≤ 120°.

7. The suture of any one of claims 1 to 6, wherein the first cam assembly (4) and the second cam assembly (5) are symmetric about a midpoint of the elongated body (1).

8. The suture of any one of claims 1 to 7, wherein the first cam assembly (4) and the second cam assembly (5) are provided with a same number of cams; and
preferably, cams (6) of the first cam assembly (4) are equally spaced, and cams (6) of the second cam assembly (5) are equally spaced.

9. The suture of any one of claims 1 to 8, wherein the first cam assembly (4) is provided with four cams;
preferably, the cams (6) of the first cam assembly (4) are spaced apart at intervals of 3 mm to 10 mm;
preferably, the second cam assembly (5) is provided with four cams; and
preferably, the cams (6) of the second cam assembly (5) are spaced apart at intervals of 3 mm to 10 mm.

10. The suture of any one of claims 1 to 9, wherein the cam (6) is secured to the elongated body (1) through heat sealing and/or the cam (6) is limited by the elongated body (1) through a stopper.

11. The suture of any one of claims 1 to 10, wherein the elongated body (1) is a single-stranded silk thread.

12. The suture of any one of claims 1 to 11, wherein the elongated body (1) and the cam assembly (2) are each made of a degradable material.

13. The suture of any one of claims 1 to 12, wherein the degradable material comprises at least one of a synthetic degradable polymer material or a natural degradable polymer material, wherein
preferably, the synthetic degradable material polymer material comprises any one or a combination of at least two of polylactic acid, L-polylactic acid, DL-polylactic acid, poly(lactic-co-glycolic acid) copolymer, polycaprolactone, poly(3-hydroxybutyrate-co-3-hydroxyvalerate), polyacetylglutamic acid, polyorthoester, polyethylene oxide/polybutene copolymer and poly(lactide-co-carpolactone) copolymer; and
preferably, the natural degradable polymer material comprises any one or a combination of at least two of collagen, gelatin, chitosan, hyaluronic acid, sodium alginate and agarose.

14. A suturing apparatus, comprising the suture of any one of claims 1 to 13 and metal needles at two ends of the suture.

15. Application of the suturing apparatus of claim 14 in tissue suturing.
